# EUROPEAN PATENT APPLICATION

(11) **EP 0 732 102 A2**
(43) Date of publication of application: **18.09.1996**
(21) Application number: 96103821.3
(22) Date of filing: 12.03.1996
(51) Int. Cl.: A61K 31/58

(54) **Composite for the treatment of acute myocardial infarction containing hirudin and acetylsalicyclic acid**

(30) Priority: 15.03.1995 US 405269; 12.05.1995 US 440556
(71) Applicant: BEHRINGWERKE Aktiengesellschaft, 35001 Marburg (DE); BRIGHAM AND WOMEN'S HOSPITAL, Boston, Massachusetts 02115 (US)
(72) Inventor: Heinrichs, Hubert, Dr., 35091 Cölbe (DE); Hennekens, Charles H., Boston, MA 02215 (US)
(74) Representative: Lauppe, Hans Friedrich

(57) **Abstract**

The invention relates to the discovery that the administration of a combination of hirudin and acetylsalicylic Acid (ASA) to patients not undergoing treatment with any thrombolytic agent, inhibits and/or prevents myocardial or cardiovascular events in the patient. The invention further relates to the pharmaceutical combination of a platelet inactivating drug, acetylsalicylic acid, and a direct thrombin inhibitor, hirudin, for the treatment of patients with acute myocardial infarction who are not undergoing thrombolysis. A pharmaceutical composition comprising the synergistic combination of hirudin and ASA, in the absence of any thrombolytic agent, is particularly useful in the therapeutic and prophylactic methods of the invention.

SEQUENCE LISTING

(1) GENERAL INFORMATION:

(i) APPLICANT:
(A) NAME: Behringwerke Aktiengesellschaft
(B) STREET: Emil-von-Behring-Str. 76
(C) CITY: Marburg
(E) COUNTRY: Deutschland
(F) POSTAL CODE (ZIP): 35041
(G) TELEPHONE: 06421-39-2205
(H) TELEFAX: 06421-39-4558

(ii) TITLE OF INVENTION: Method of treating acute myocardial
infarction with hirudin and acetylsalicylic acid in
patients not undergoing thrombolytic treatment

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(vi) PRIOR APPLICATION DATA:
(A) APPLICATION NUMBER: US 08/405,269
(B) FILING DATE: 15-MAR-1995

(vi) PRIOR APPLICATION DATA:
(A) APPLICATION NUMBER: US 08/440,556
(B) FILING DATE: 12-MAY-1995

(2) INFORMATION FOR SEQ ID NO: 1:

(i) SEQUENCE CHARACTERISTICS:
(A) LENGTH: 65 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(vi) ORIGINAL SOURCE:
(A) ORGANISM: Hirudo medicinalis

(xi) SEQUENCE DESCRIPTION: SEQ ID NO: 1:

Val Val Tyr Thr Asp Cys Thr Glu Ser Gly Gln Asn Leu Cys Leu Cys
1               5                   10                  15

Glu Gly Ser Asn Val Cys Gly Gln Gly Asn Lys Cys Ile Leu Gly Ser
            20                  25                  30

Asp Gly Glu Lys Asn Gln Cys Val Thr Gly Glu Gly Thr Pro Lys Pro
        35                  40                  45

Gln Ser His Asn Asp Gly Asp Phe Glu Glu Ile Pro Glu Glu Tyr Leu
    50                  55                  60

Gln
65

(2) INFORMATION FOR SEQ ID NO: 2:

(i) SEQUENCE CHARACTERISTICS:
(A) LENGTH: 65 amino acids
(B) TYPE: amino acid
(C) STRANDEDNESS: single
(D) TOPOLOGY: linear

(ii) MOLECULE TYPE: protein

(iii) HYPOTHETICAL: YES

(iv) ANTI-SENSE: NO

(vi) ORIGINAL SOURCE:
(A) ORGANISM: Hirudo medicinalis

(xi) SEQUENCE DESCRIPTION: SEQ ID NO: 2:

Ile Thr Tyr Thr Asp Cys Thr Glu Ser Gly Gln Asn Leu Cys Leu Cys
1               5                   10                  15

Glu Gly Ser Asn Val Cys Gly Lys Gly Asn Lys Cys Ile Leu Gly Ser
            20                  25                  30

Asn Gly Lys Gly Asn Gln Cys Val Thr Gly Glu Gly Thr Pro Asn Pro
        35                  40                  45

Glu Ser His Asn Asn Gly Asp Phe Glu Glu Ile Pro Glu Glu Tyr Leu
    50                  55                  60

Gln
65

(2) INFORMATION FOR SEQ ID NO: 3:

(i) SEQUENCE CHARACTERISTICS:
(A) LENGTH: 66 amino acids
(B) TYPE: amino acid
(C) STRANDEDNESS: single
(D) TOPOLOGY: linear

(ii) MOLECULE TYPE: protein

(iii) HYPOTHETICAL: YES

(iv) ANTI-SENSE: NO

(vi) ORIGINAL SQURCE:
(A) ORGANISM: Hirudo medicinalis

(xi) SEQUENCE DESCRIPTION: SEQ ID NO: 3:

Ile Thr Tyr Thr Asp Cys Thr Glu Ser Gly Gln Asn Leu Cys Leu Cys
1               5                   10                  15

Glu Gly Ser Asn Val Cys Gly Lys Gly Asn Lys Cys Ile Leu Gly Ser
            20                  25                  30

Gln Gly Lys Asp Asn Gln Cys Val Thr Gly Glu Gly Thr Pro Lys Pro
        35                  40                  45

Gln Ser His Asn Gln Gly Asp Phe Glu Pro Ile Pro Glu Asp Ala Tyr
    50                  55                  60

Asp Glu
65

## Description

### Background of the Invention

### Field of the Invention

The invention relates to the therapeutic and pharmaceutical combination of a platelet inactivating drug, acetylsalicylic acid, and a direct thrombin inhibitor, hirudin, for the treatment of patients with acute myocardial infarction who are not undergoing thrombolysis.

### Description of Related Art

### 1. Acute Vascular Diseases and Clot Formation

Acute vascular diseases, such as myocardial infarction, stroke, pulmonary embolism, deep vein thrombosis, peripheral arterial occlusion and other blood system thromboses constitute major health risks. In particular, acute myocardial infarction (AMI) occurs with a mortality rate of about 35%, with about half of the deaths occurring before the patient reaches a hospital; an additional 15 to 20% of survivors die in the first year following myocardial infarction (Pasternak, R.C., *et al.,* Chapter 190 in *Harrison's Principles of Internal Medicine*, 11th ed., Braunwald *et al*., Eds., McGraw-Hill Book Co., New York (1987), pages 982-993). Acute vascular diseases are caused or aggravated by either partial or total occlusion of a blood vessel by a blood clot, which typically consists of fibrin and/or aggregated platelets.

Blood platelets play an essential role in normal hemostasis. They have both a distinct hemostatic function and a thromboplastic function. Hemostasis is initiated within a few seconds following a trauma, when platelets begin to adhere to the edges of the lesion. This initial adherence of platelets may be mediated by collagen exposed at the site of blood vessel wall trauma or by newly generated thrombin. Once in contact with collagen or thrombin, platelets undergo activation (release reaction) and release a variety of chemicals, including ADP and thromboxane A2. The released ADP and thromboxane A2 cause additional platelets from the issuing blood to aggregate to those already attached to the vessel wall. The newly attached platelets also undergo the release reaction and the process continues until a hemostatic platelet plug forms. In addition to releasing ADP and thromboxane A2, platelets also expose platelet factor 3, which promotes the clotting cascade, ultimately resulting in thrombin generation and fibrin deposition at the site of injury. Thrombin also binds to receptors on the platelet membrane and causes further platelet aggregation and release. The ultimate result is a mixed clot composed of aggregated platelets and polymerized fibrin (Handin, R.I., Chapter 154 in *Harrison's Principles of Internal Medicine*, 11th ed., Braunwald *et al*., Eds., McGraw-Hill Book Co., New York (1987), pages 266-272).

Although necessary for normal hemostasis, clot formation resulting from platelet aggregation and release reaction is now recognized as being responsible for, or contributing to, a variety of life-threatening vascular diseases, such as myocardial infarction, stroke, peripheral arterial occlusion and other blood system thromboses. In patients suffering from such diseases, clot formation is an undesirable event, and inhibiting or controlling clot formation is prophylactic or therapeutic for such patients.

### 2. Acetylsalicylic Acid

Commonly known as aspirin, acetylsalicylic acid is commonly described as an analgesic (see, e.g., p. 16 of *The Merck Index*, 11th Ed., Budavari *et al*., eds., Merck & Co., Rahway, NJ (1989)). Recently, however, ASA has been investigated for use as a therapeutic anticoagulant, although the degree of therapeutic value of ASA treatment for specific cardiovascular conditions remains a subject of controversy (Pasternak, R. C., *et al*., Chapter 190 in *Harrison's Principles of Internal Medicine*, 11th ed., Braunwald *et al*., Eds., McGraw-Hill Book Co., New York (1987), page 987; Aldhous, P., *Science 263:24* (1994)). ASA is synthesized by known methods (e.g., German Patent No. 236,196 (1908)).

### 3. Acetylsalicylic Acid in Patients with Acute Myocardial Infarction

As shown in a large, double-blind, randomized and placebo-controlled trial, the use of acetylsalicylic acid (ASA) reduces mortality, reinfarctions and strokes in patients with acute myocardial infarction (AMI). Specifically, in the Second International Study of Infarct Survival (ISIS-2), performed on 17,187 patients with AMI treated within 24 hours of the onset of symptoms, daily treatment with 162.5 mg of ASA reduced the 35-day mortality by 23% (ISIS-2 Collaborative Group, *Lancet ii* 349-360 (1988)). In addition, the incidence of nonfatal reinfarctions decreased by 49% and of nonfatal stroke by 46%. There was no increase in major bleeds (defined as bleeds requiring transfusions) (i.e., 31% with ASA versus 33% with placebo), and only a modest increase (0.6%) of minor bleeds (ISIS-2 Collaborative Group, *Lancet ii*:349-360 (1988)). The beneficial effects of ASA were observed in AMI patients undergoing thrombolysis, as well as in those not receiving thrombolysis

### 4. Heparin

Heparin is a complex linear water soluble polysaccharide of 60 to 100 kDa that is comprised of a variety of oligosaccharide structures. The use of heparin to prevent the *in vitro* clotting of blood removed from the body, eventually led to its *in vivo* use to treat venous thromboembolism, in which thrombin activity is responsible for the development or expansion of a thrombus (Majerus, P.W., *et al*., Chapter 55 in *Goodman and Gilman's The Pharmacological Basis of Therapeutics,* 8th Ed., Pergamon Press, NY (1990)).

Heparin is an indirect thrombin inhibitor which acts by increasing, by at least 1,000-fold, the rate at the inhibition of thrombin by antithrombin III, a member of the "serpin" family of serine protease inhibitors (Olson, S.T., *J. Biol. Chem*. 263:1698-1708 (1988); Tollefsen, D.M., pages 257-273 in *Heparin: Chemical and Biological Properties, Clinical Applications,* Lane, D.A., *et al.*, eds., Edward Arnold Publishers, Ltd., London (1989)). Recently, it has become clear that heparin has additional activities *in vivo* including but not limited to (1) increasing the rate at which HCII (heparin cofactor II), another member of the "serpin" family of serine protease inhibitors, inhibits thrombin; (2) promoting the binding of thrombin to fibrin polymers (Derechin, V.M., *et al., J. Biol. Chem.* 265:5623-5638 (1990); Hogg, P.J., *et al., J. Biol. Chem.* 265:241-247 (1990)).

Unfractionated Heparin is widely used in AMI patients as adjunctive antithrombotic therapy. Because of its mode of action, heparin is useful to prevent only thrombin-induced platelet aggregation. Even in that application, however, the overall efficacy of heparin is questionable. Furthermore, therapy with heparin is limited by several factors, including but not limited to the following.

First, heparin produces many undesirable side effects, including hemorrhaging, heparin-induced thrombocytopenia, abnormalities in hepatic function, osteoporosis, hyperkalemia and the like (Majerus, P.W., *et al.*, Chapter 55 in *Goodman and Gilman's The Pharmacological Basis of Therapeutics*, 8th Ed., Pergamon Press, NY (1990)).

Second, many blood proteins, including platelet factor 4, secreted by activated platelets, and lactoferrin, secreted by activated neutrophils, inactivate heparin. Histidine-rich glycoprotein and S-protein (vitronectin) also competitively inhibit the binding of antithrombin to heparin (Majerus, P.W., *et al*., Chapter 55 in *Goodman and Gilman's The Pharmacological Basis of Therapeutics,* 8th Ed., Pergamon Press, NY (1990)). Due to a variable degree of protein binding and heparin inactivation, the dosing of heparin has to be individually adjusted for each patient, and the clotting time (i.e., the activated partial thromboplastin time (aPTT)) of the patient's blood must be closely monitored during the course of treatment (Majerus, P.W., *et al*., Chapter 55 in *Goodman and Gilman's The Pharmacological Basis of Therapeutics,* 8th Ed., Pergamon Press, NY (1990)).

Third, immunological responses limit heparin use in some patients. In about 5 to 10% of patients treated with heparin for more than 5 days intravenously, antibodies are induced. In about 0.2 to 0.5% of patients treated with unfractionated heparin, the heparin induced antibodies result in thrombocytopenia and severe thromboembolic complications. In patients suffering from heparin-induced thrombocytopenia, immune-mediated thrombocytopenia may have dire thrombotic consequences, as heparin actually accelerates platelet aggregation, often with fatal consequences.

### 5. Heparin and ASA for Treatment of AMI Patients

Despite the fact that heparin is widely used in AMI, evidence for its net benefits in patients receiving an adequate dose of ASA is sparse. In the ISIS-2 trial, the effects of the thrombolytic agent, streptokinase, alone or in combination with ASA, and ASA alone were compared against placebo in a two-by-two factorial design. The trial provided conclusive evidence of the beneficial effects of thrombolytic therapy with streptokinase, and of antithrombotic therapy with ASA, with the benefits being largely additive (ISIS-2 Collaborative Group, *Lancet ii*:349-360 (1988)). Furthermore, in subgroup analyses, patients receiving heparin in addition to ASA were exploratively compared with patients receiving only ASA. No increased clinical benefit due to the additional use of heparin was demonstrable.

Other studies have suggested that heparin treatment might have negative effects when used in combination with ASA. In ISIS-3 (ISIS-3 Collaborative Study Group, *Lancet* 339:753-770 (1992)), 41,299 patients ware treated with ASA, thrombolytic drugs (i.e., streptokinase, recombinant tissue Plasminogen Activator (rt-PA), or anisoylated-plasminogen-streptokinase activator complex (APSAC)), and either (1) subcutaneous heparin started four hours after randomization or (2) no heparin. The daily dose of ASA was 162.5 mg. The 35-day mortality in the group treated with heparin (10.3%) was similar to that of the group without heparin (10.6%). However, the incidence of probable cerebral hemorrhages or cerebral hemorrhages was significantly higher in the group treated with heparin. The GISSI-2 study (Groppo Italian per lo Studio della Sopravivenza Nell'Infarto Micardico, *Lancet* 83:65-71 (1990)) showed similar results. Overall, there was no significant benefit with regard to mortality, but a 2 per 1,000 increase in risk of cerebral hemorrhage.

The Global Utilization of Streptokinase and Tissue Plasminogen Activator to Treat Occluded Arteries (GUSTO-1) trial (The GUSTO Investigators, *N. Engl. J. Med.* 329:673-682 (1993)) compared different thrombolytic agents (i.e., streptokinase and rt-PA) and the adjunctive therapy of immediate intravenous or delayed subcutaneous therapy with heparin in those who received streptokinase. All patients received a sufficient dose of ASA. There was no difference in benefit of heparin between the two modes of administration, i.e., intravenously (I.V.) and subcutaneously (S.C.), as adjunctive therapy to streptokinase. The 30-day mortality in the group receiving heparin I.V. (7.4%) was comparable to that in the group receiving heparin S.C. (7.2 %). Furthermore, there was no evidence for less refarction, stroke, or bleeding with immediate I.V. heparin.

Overall, the available data are inconclusive with regard to a net clinical benefit of heparin in AMI patients treated with ASA (Ridker *et al.,* Lancet 341:1574-1577 (1993); Fibrinolytic Therapies Trialists collaborative Group, *Lancet* 343:311-322 (1994)), whether or not the patients received thrombolytic therapy.

### 6. Hirudin and Variants Thereof

Hirudin is a family of related polypeptides, each of about 65 amino acids in length, the first member of which was isolated from the leech *Hirudo medicinalis* (Markwardt, F., *Methods Enzymol.* 19:924-932 (1970)). At least two different isospecific forms of hirudin, HV-1 and HV-2, have been sequenced and have been shown to differ slightly in amino acid sequence (Harvey, R.P., *et al., Proc. Natl. Acad. Sci.* (USA) 83:1084-1088 (1986)). Both HV-1 and HV-2 comprise a single polypeptide chain protein containing 65 amino acids in which the amino terminus primarily comprises hydrophobic amino acids and the carboxyl terminus typically comprises polar amino acids. Sequence analysis of naturally occurring forms of hirudin revealed homologies of about 80%, and six cysteine residues were assigned to three disulfide bonds connecting Cys⁶ to CyS₁₄, CyS₁₆ to CyS₂₈ , and CyS₂₂ to CYS³⁹ (Dodt, J., *et al., Biol. Chem. Hoppe-Seyler* 366:379-385 (1985); Mao, S.J.T., *et al., Anal. Biochem.* 161:514-518 (1987); Harvey, R.P., *et al., Proc. Natl. Acad. Sci.* (USA) 83:1084-1088 (1986)). All forms of hirudin are characterized by an N-terminal domain (residues 1-39) stabilized by the three disulfide bridges, and a highly acidic C-terminal segment (residues 40-65) characterized by the presence of a tyrosine residue at amino acid position 63 which is sulfated. The three-dimensional structure of hirudin in solution has been determined (Clore, G.M., *et al., EMBO J.* 6:529-537 (1987); Haruyama *et al., Biochemistry* 28:4301-4312 (1989)).

The amino acid sequences of three hirudin varients are shown below:
(a) hirudin variant HV1 has the formula 
Val Val Tyr Thr Asp Cys Thr Glu Ser Gly
Gln Asn Leu Cys Leu Cys Glu Gly Ser Asn
Val Cys Gly Gln Gly Asn Lys Cys Ile Leu
Gly Ser Asp Gly Glu Lys Asn Gln Cys Val
Thr Gly Glu Gly Thr Pro Lys Pro Gln Ser
His Asn Asp Gly Asp Phe Glu Glu Ile Pro
W
|
Glu Glu Tyr Leu Gln,
 wherein W represents the phenolic hydroxy group of Tyr (desulfato hirudin HVI) or a group of the formula -O-SO₃H (hirudin HV1),
(b) hirudin variant HV2 has the formula
   Ile Thr Tyr Thr Asp Cys Thr Glu Ser Gly Gln Asn Leu Cys Leu Cys Glu Gly Ser Asn Val Cys Gly Lys Gly Asn Lys Cys Ile Leu Gly Ser Asn Gly Lys Gly Asn Gln Cys Val Thr Gly Glu Gly Thr Pro Asn Pro Glu Ser His Asn Asn Gly Asp Phe Glu Glu Ile Pro Glu Glu Tyr Leu Gln,
   and,
(c) hirudin variant PA has the formula
   Ile Thr Tyr Thr Asp Cys Thr Glu Ser Gly Gln Asn Leu Cys Leu Cys Glu Gly Ser Asn Val Cys Gly Lys Gly Asn Lys Cys Ile Leu Gly Ser Gln Gly Lys Asp Asn Gln Cys Val Thr Gly Glu Gly Thr Pro Lys Pro Gln Ser His Asn Gln Gly Asp Phe Glu Pro Ile Pro Glu Asp Ala Tyr Asp Glu.

Hirudin is a direct thrombin inhibitor with a very high affinity for human thrombin (Stone, S.R., *et al., Biochemistry* 25:4622-4628 (1986)) as well as thrombin from other mammalian species (Dodt, J., *et al*., *FEBS Lett.* 229:87-90 (1988)). One molecule of hirudin binds to one molecule of thrombin, forming a tight, noncovalent complex and thereby inactivating thrombin essentially irreversibly, i.e., the conversion of fibrinogen to fibrin and the thrombin-mediated activation of platelets is blocked. In contrast to heparin, hirudin also inactivates clot-bound thrombin (Weitz *et al., J. Clin. Invest.* 86:385-391 (1990)). No antithrombin III or any other cofactor is required for inactivation of thrombin. Hirudin is not inactivated by platelet factor 4, and does not induce thrombocytopenia or severe thromboembolic complications (Markwardt, F., *et al., Thromb. Haemostasis* 52:160-163 (1984); Talbot, M., *Semin. Thromb. Hemost.* 15:293-301 (1989); Prager, N.A., *et al., J. Am. Coll. Cardiol.* 22:296-301 (1993)). In animal studies, hirudin exhibits low toxicity, little or no antigenicity and a very short clearance time from circulation (Markwardt, F., *et al., Thromb. Haemostasis* 47:226-229 (1982); Heras, M., *et al., Circulation* 79:657-665 (1989)).

In comparison to heparin, plasma levels of hirudin are much more stable and reproducible, as protein binding is rather low; as a result, there is less interindividual variety of treatment for hirudin as contrasted to heparin (Badimon, L., *et al., Trends Cardiovasc. Med.* 1:261-267 (1991)). In several pilot studies in AMI patients undergoing thrombolysis, treatment with recombinant hirudin in conjunction with ASA has shown some promising results (Neuhaus *et al., J. Am. Coll. Cardiol.* 21:418A (1993); von Essen *et al., Ann. Hematol. 68 (Suppl. II)*:A43 (1994); Cannon *et al., J. Am. Coll. Cardiol.* 23:993-1003 (1994)). Treatment of patients having unstable angina with recombinant hirudin in combination with ASA has also been examined (Topol, E.J., *et al., Circulation* 89:1557-1566 (1994)).

Methods for the preparation and biological or biochemical evaluation of various natural forms of hirudin, as well as many variants of hirudin (a.k.a. "hirulogs"), have been described. In an effort to create a greater supply of hirudin, attempts have been made to produce the polypeptide through recombinant DNA techniques. The presence of an O-sulfated tyrosine residue on native hirudin and the inability of microorganisms to perform a similar protein modification made the prospect of recombinant production of biologically active hirudin highly speculative. However, desulfato-hirudins are almost as active as their sulfated counterparts (U.S. Patent Nos. 4,654,302 (1987) and 4,801,576 (1989)), and recombinant (i.e., desulfato-) hirudin has been produced in *Escherichia coli* (European patent application Nos. 158,564 (1985), 168,342 (1986), 171,024 (1986), 448,093 (199 1), and 506,883 (1994)) and yeast (European patent application Nos. 200,655 (1986) and 252,854 (1988)). Furthermore, methods are known in the art for effecting the O-sulfation, or comparable modifications, of recombinant hirudin *in vitro* (U.S. Patent Nos. 225,633 (1987), 4,745,177 (1988), and 5,256,559 (1993)).

Efforts have been made to identify variants of hirudin, including peptide fragments of hirudin, which are effective in inhibiting clotting. For example, hirudin-PA and its derivatives are structurally very similar to hirudin; however, at several important places on the protein chain, hirudin-PA and its derivatives differ in a characteristic manner from the sequence of hirudin. In hirudin-PA the strongly acid sulfate monoester group on the phenolic hydroxyl of the tyrosine group in position 64 (position 63 in hirudin) is conspicuous (U.S. Patent No. 4,767,742 (1988)). Oligopeptides and peptidomimetic analogs derived from hirudin, as well as mutant forms of hirudin, having anticoagulant and platelet inhibitory effects are known in the art (U.S. Patent Nos. 5,256,559 (1993), 4,654,302 (1987), and 4,668,662 (1987); European Patent Appl. No. 576,792 (1994); International Patent Appl. Nos. WO 86/03517, WO 92/08736 (1992), and WO 94/080034 (1994)). Furthermore, methods are known in the art for producing soluble, biocompatible compositions comprising hirudins (U.S. Patent No. 5,112,615 (1992)).

Thus, prior to the present invention, a need existed for an adjunctive antithrombotic therapy for AMI that utilizes a thrombin inhibitor that does not have the undesirable characteristics of heparin.

### Incorporation by Reference

The entire text of all publications cited above and below are hereby incorporated by reference.

### Summary of the Invention

We have discovered that the administration of the combination of hirudin and ASA to patients not undergoing treatment with any thrombolytic agent, inhibits and/or prevents myocardial or cardiovascular events in the patient treated thereby. Myocardial or cardiovascular events include, but are not limited to, myocardial infarctions of various degrees of severity, strokes, coronary revascularization and cardiovascular death. In particular, we have discovered that the administration of the combination of hirudin and ASA to patients is effective in preventing the recurrence of acute myocardial infarctions (AMI).

The invention is also directed to a pharmaceutical composition comprising the synergistic combination of hirudin and ASA, in the absence of any thrombolytic agent. The pharmaceutical composition of the invention is particularly useful in the therapeutic and prophylactic methods of the invention.

### Detailed Description of the Preferred Embodiments

A preferred method of the invention includes the administration of ASA (aspirin) in combination with one or more variants or derivatives of hirudin to a patient such that the occurrence of one or more myocardial or cardiovascular events is thereby inhibited and/or prevented, wherein the patient being treated is not undergoing treatment with any thrombolytic agent. The term "myocardial or cardiovascular events" is meant to include, but not be limited to, myocardial infarctions of various degrees of severity, strokes, coronary revascularization and cardiovascular death. By the term "inhibited and/or prevented" is intended the prevention, amelioration, or both, of such events. Thus, the invention includes both a therapeutic and a prophylactic modality.

Included as well in the present invention are pharmaceutical compositions comprised of ASA and hirudin in combination with a pharmaceutically acceptable carrier. Because the compositions of the invention are intended for treatment of patients who are not undergoing treatment with any thrombolytic agent, such agents are excluded from the composition of the invention. The term "thrombolytic agent" includes streptokinase, urokinase, tissue Plasminogen Activator (t-PA or, when produced via recombinant DNA technology, rt-PA), anisoylated-Plasminogen-Streptokinase activator complex (APSAC), and the like (Majerus, P.W., *et al*., Chapter 55 in *Goodman* *and Gilman's The Pharmacological Basis of Therapeutics,* 8th Ed., Pergamon Press, NY (1990)).

By the term "ASA" is intended acetylsalicylic acid, also known as aspirin, 2-(acetyloxy)benzoic acid 2-carboxyphenyl ester, 2-hydroxybenzoic acid acetate 2-carboxyphenyl aster, salicylacetylic acid, and Displosal Acetate. Also included by the term "ASA" are derivatives thereof having the biological activity of ASA.

The dosage of ASA varies according to different embodiments of the invention, as well as the age, size and medical condition of the patient. Thus, in one embodiment, the dose of ASA does not vary over the period of treatment and is about 50 to about 500 mg/day, in particular about 80 to about 300 mg/day, and preferably about 80 to about 100 mg/day. In another embodiment, the dose of ASA is about 160 to about 325 mg/day for the first 1 to 5 days following an AMI, and is about 80 to about 160 mg/day for on subsequent days. In all embodiments, ASA is preferably administered orally, and, in therapeutic modalities, is preferably administered beginning as soon as possible after a diagnosis of AMI has been made and continuing for up to about 3 to 6 months after the event.

By the term "hirudin" is intended hirudin family members isolated from their natural sources; recombinant hirudin, produced by isolating a gene encoding a hirudin protein and introducing the gene into an appropriate vector/host system for production of hirudin; and synthetic oligopeptides and peptidomimetics derived from hirudin having the anticoagulant activity of hirudin. There are many *in vitro* and *in vivo* methods known in the art for determining the anticoagulant activity of a particular hirudin. For example, *in vitro* tests include (1) determinations of inhibition of platelet aggregation, thrombin-induced or otherwise, by a hirudin variant (often as measured by the activated partial generation, and/or (3) determinations of inhibition of thrombin's enzymatic activity on chromogenic substrates such an Chromozym TH (U.S. Patent Nos. 5,256,559 (1993), 5,246,715 (1993), 5,242,810 (1993), 5,227,469 (1993), 4,832,849 (1989), and 4,801,576 (1989)). The term "hirudin" includes, but is not limited to, hirudin HV1, desulfato hirudin, (Ile^{1,2})-desulfato hirudin HV1, (Gly¹)-desulfato hirudin HV1, (Gln^{57,58,61,62})-desulfato hirudin HV1, (Pro⁶⁶)-desulfato hirudin HV1, (Gln²⁷, Arg⁴⁷)-desulfato hirudin HV1, (Met⁰, Gln²⁷, Arg⁴⁷)-desulfato hirudin HV1, (Gln²⁷, Gln³⁶, Arg⁴⁷)-desulfato hirudin HV1, (des-Val¹, Thr²)-desulfato hirudin HV1, (Leu¹, Thr²)-desulfato hirudin HV1, hirudin HV2, (Lys⁴⁷)-hirudin HV2, and hirudin PA.

The dosage of hirudin, and means of administration thereof, vary according to different embodiments of the invention, as well as the age, size and medical condition of the patient. Thus, in one embodiment, the dose of hirudin does not vary over time and is 0.05 to 0.4 mg/kg body weight/day, preferably 0.1 to 015 mg/kg body weight/day, and is administered intravenously. In a related embodiment, the dose of hirudin does not vary over time and is 0.25 to 1.0 mg/kg body weight/12 hours, preferably about 0.5 mg/kg body weight/12 hours, and is administered subcutaneously. In another embodiment, an initial dose of hirudin in 0.2 to 0.4 mg/kg body weight administered intravenously in a bolus followed by a current infusion of 0.05 to 0.2 mg/kg body weight/day of hirudin, preferably 0.1 to 0.15 mg/kg body weight/day hirudin. In all embodiments, hirudin is preferably administered orally, and, in therapeutic modalities, is preferably administered beginning as soon as possible after a diagnosis of AMI has been made and continuing for up to about 3 to 6 months after the event.

By the term "patients not undergoing thrombolytic treatment" is intended those patients suffering from AMI

By the term "patients not undergoing thrombolytic treatment" is intended those patients suffering from AMI for whom thrombolytic therapy is not available due to the presence of one or more medical contraindications or for any other reason. Medical contraindications to thrombolytic therapy include, but are not limited to, (1) surgery (e.g., organ biopsy, puncture of noncompressible vessels, serious trauma or cardiopulmonary resuscitation) within 10 days, (2) serious gastrointestinal bleeding within three months, (3) history of hypertension, (4) active bleeding or hemorrhagic disorder and (5) previous cerebrovascular accident or active intracranial process) (Majerus, P.W., *et al*., Chapter 55 in *Goodman and Gilman's The Pharmacological Basis of Therapeutics,* 8th Ed., Pergamon Press, NY (1990)). Additionally, patients may elect to not pursue thrombolytic therapy for religious or other personal reasons. By the terms "thrombolytic therapy" and "thrombolytic treatment" is meant the administration of one or more thrombolytic agents to a patient in need thereof. Thrombolytic agents include, but are not limited to, streptokinase, urokinase, tissue Plasminogen Activator (t-PA or, when produced via recombinant DNA technology, rt-PA), anisoylated-Plasminogen-Streptokinase activator complex (APSAC), and the like (Majerus, P.W., *et al*., Chapter 55 in *Goodman and Gilman's The Pharmacological Basis of Therapeutics,* 8th Ed., Pergamon Press, NY (1990)).

This invention is also directed to the use of hirudin in combination with ASA in AMI patients not undergoing thrombolysis. Currently, more than 50% of all AMI patients in the U.S.A. and a variable proportion of AMI patients in Europe (from 30% in UK to about 60 to 70% in France) are not treated by thrombolysis. The reasons for this can be, e.g., a long delay from onset of symptoms to treatment, an uncharacteristic ECG, old age, uncontrolled hypertension, recent stroke, or gastroduodenal ulcer.
For this patient group it has been shown that mortality rates and the incidence of reinfarction and of other clinical complications are higher than in patients undergoing thrombolysis.

At present it is established that ASA confers clinical benefit to these patients via the partial inactivation of platelets. The additional use of hirudin with the inactivation of thrombin is expected to result in additional net clinical benefit resulting in a reduction of mortality or reinfarctions, as well as other clinical complications.

The results of several trials raise the possibility that the combination of recombinant hirudin and ASA with differential antithrombotic mode of action is superior to the combination of heparin and ASA or to ASA alone. Recently it has been demonstrated that adjunctive antithrombotic therapy with recombinant hirudin in addition to thrombolysis with rt-PA or streptokinase resulted in a higher incidence of early, complete and sustained patency of the infarct-related coronary artery. In other clinical studies it was demonstrated that a higher incidence of early and complete patency correlates with a reduced mortality (Neuhaus *et al., J. Am. Coll. Cardiol.* 21:418A (1993); von Essen *et al., Ann. Hematol. 68 (Suppl. II)*:A43 (1994); Cannon *et al., J. Am. Coll. Cardiol.* 23:993-1003 (1994)). In addition it has been demonstrated that open vessels are important also for AMI patients not undergoing thrombolysis (GUSTO Angiographic Investigators, *N. Engl. J. Med.* 329-1615-1622 (1993); Vogt *et al., J. Am. Coll. Cardiol*. 21:1391-1395 (1993)).

Currently, the need of improved therapy of AMI patients who cannot be treated by thrombolysis has not been specifically addressed in preclinical models, which only focused on, e.g., reocclusions. No clinical study addresses the question of how to improve treatment of AMI patients with Q-wave infarctions not treated by thrombolysis. The ongoing GUSTO-2b study assesses the treatment of patients with non-Q wave myocardial infarction in a subgroup of patients with acute coronary syndromes. This study will not definitively answer the question of optimal treatment of patients with AMI not undergoing thrombolysis, as only a specific subgroup of these patients are assessed.

We have observed that the combined use of hirudin and ASA in AMI patients where treatment with thrombolysis is not advisable will result in a higher incidence of open coronary vessels. In addition, spontaneous reperfusion via the endogenous thrombolytic system will be increased by the use of hirudin. This innovative and original combination of platelet inhibition and direct thrombin inhibition results in net clinical benefit in AMI patients not undergoing thrombolytic treatment.

## Claims

1. A method for the preparation of a pharmaceutical composition for treating or preventing acute myocardial infarction in a patient, comprising ASA and hirudin.

2. The method of claim 1, wherein said ASA and said hirudin are administered in separate unit dosage forms.

3. The method of claim 1, wherein said ASA and said hirudin are together in a single unit dosage form.

4. The method of claim 1, wherein said patient is not being treated with a thrombolytic agent.

5. The method of claim 1, wherein said hirudin is selected from the group consisting of
(a) hirudin variant HV1 having the formula 
Val Val Tyr Thr Asp Cys Thr Glu Ser Gly
Gln Asn Leu Cys Leu Cys Glu Gly Ser Asn
Val Cys Gly Gln Gly Asn Lys Cys Ile Leu
Gly Ser Asp Gly Glu Lys Asn Gln Cys Val
Thr Gly Glu Gly Thr Pro Lys Pro Gln Ser
His Asn Asp Gly Asp Phe Glu Glu Ile Pro
W
|
Glu Glu Tyr Leu Gln,
 wherein W represents the phenolic hydroxy group of Tyr (desulfato hirudin HVI) or a group of the formula -O-SO₃H (hirudin HV1),
(b) hirudin variant HV2 having the formula
Ile Thr Tyr Thr Asp Cys Thr Glu Ser Gly Gln Asn Leu Cys Leu Cys Glu Gly Ser Asn Val Cys Gly Lys Gly Asn Lys Cys Ile Leu Gly Ser Asn Gly Lys Gly Asn Gln Cys Val Thr Gly Glu Gly Thr Pro Asn Pro Glu Ser His Asn Asn Gly Asp Phe Glu Glu Ile Pro Glu Glu Tyr Leu Gln,
and
Ile Thr Tyr Thr Asp Cys Thr Glu Ser Gly Gln Asn Leu Cys Leu Cys Glu Gly Ser Asn Val Cys Gly Lys Gly Asn Lys Cys Ile Leu Gly Ser Gln Gly Lys Asp Asn Gln Cys Val Thr Gly Glu Gly Thr Pro Lys Pro Gln Ser His Asn Gln Gly Asp Phe Glu Pro Ile Pro Glu Asp Ala Tyr Asp Glu.

6. The method of claim 1, wherein said hirudin is selected from the group consisting of (Ile^{1,2})-desulfato hirudin HV1, (Gly¹)-desulfato hirudin HV1, (Gln^{57,58,61,62})-desulfato hirudin HV1, (Pro⁶⁶)-desulfato hirudin HV1, (Gln²⁷, Arg⁴⁷)-desulfato hirudin HV1, (Met⁰, Gln²⁷, Arg⁴⁷)-desulfato hirudin HV1, (Gln²⁷, Gln³⁶, Arg⁴⁷)-desulfato hirudin HV1, (des-Val¹, Thr²)-desulfato hirudin HV1, (Leu¹, Thr²)-desulfato hirudin HV1 and (Lys⁴⁷)-hirudin HV2.

7. The method of claim 1, wherein a human or animal patient is treated.

8. The method of claim 1, wherein said ASA is given orally.

9. The method of claim 8, wherein said ASA is administered in a dosage consisting of 50-500 mg/day.

10. The method of claim 8, wherein said ASA regimen is 160-325 mg/day for the first day to fifth day of treatment, and 80-100 mg/day for the succeeding days.

11. The method of claim 1, wherein said hirudin is administered intravenously or subcutaneously.

12. The method of claim 11, wherein said hirudin is administered in a dosage of 0.05-0.40 mg/kg body weight/day administered intravenously.

13. The method according to claim 11, wherein said hirudin regimen is 0.2-0.4 mg/kg body weight as an initial bolus given intravenously, followed by infusion of 0.05-0.2 mg/kg body weight/day.

14. The method of claim 11, wherein hirudin at 0.25-1.0 mg/kg body weight twice a day given subcutaneously.

15. A pharmaceutical composition comprising ASA and hirudin.

16. The pharmaceutical composition of claim 15 comprising further one or more pharmaceutically acceptable carriers.

17. The pharmaceutical composition of claim 15, wherein said pharmaceutical composition does not contain any thrombolytic agents.

18. The composition of claim 15, wherein said hirudin is selected from the group consisting of hirudin HV1, desulfato hirudin, (Ile^{1,2})-desulfato hirudin HV1, (Gly¹)-desulfato hirudin HV1, (Gln^{57,58,61,62})-desulfato hirudin HV1, (Pro⁶⁶)-desulfato hirudin HV1, (Gln²⁷, Arg⁴⁷)-desulfato hirudin HV1, (Met⁰, Gln, Arg⁴⁷)-desulfato hirudin HV1, (Gln²⁷, Gln³⁶, Arg⁴⁷)-desulfato hirudin HV1, (des-Val¹, Thr²)-desulfato hirudin HV1, hirudin HV2, (Lys⁴⁷) hirudin HV2, and hirudin PA.
